# FASCICULE DE BREVET EUROPEEN

(11) **EP 1 757 569 B1**
(45) Date de publication et mention de la délivrance du brevet: **22.02.2017**
(21) Numéro de dépôt: 06125186.4
(22) Date de dépôt: 07.03.2001
(51) Int. Cl.: G01N 30/72, C07C 19/08, C07C 17/38

(54) **Méthode d'analyse d'un hydrofluoroalcane**
Methode zur Analyse eines Hydrofluoralkan
Method for analysing a hydrofluoroalcane

(30) Priorité: 07.03.2000 FR 0002944
(43) Date de publication de la demande: 28.02.2007
(62) Demande divisionnaire de: 01929432.1
(73) Titulaire: Daikin Industries, Ltd., Osaka-shi, Osaka 530-8323 (JP)
(72) Inventeur: Balthasart, Dominique, 1120, Bruxelles (BE); Anciaux, Charles-Marie, 1180, Bruxelles (BE); Mahaut, Yves, 1600, Sint-Pieters-Leeuw (BE); Klug, Roland, 65510, Idstein (DE)
(74) Mandataire: Hoffmann Eitle

(56) Documents cités:
- EP-A- 0 389 334
- EP-A- 0 467 531
- EP-A- 0 472 391
- EP-A- 0 507 458
- EP-A- 0 508 631
- EP-A- 0 526 002
- WO-A-94/19301
- WO-A-97/37955
- WO-A-99/50208
- US-A- 4 129 603
- US-A- 5 696 310

## Description

La présente invention a pour objet une méthode d'analyse d'un hydrofluoroalcane.

Certains hydrofluoroalcanes comme notamment le 1,1,1,2-tétrafluoroéthane et le 1,1,1,2,3,3,3-heptafluoropropane sont utilisables, en raison de leurs propriétés physiques et de leur toxicologie favorable dans des applications pharmaceutiques, notamment comme gaz propulseur dans des aérosol médicaux. La production industrielle de tels hydrofluoroalcanes fournit cependant un produit qui contient des impuretés saturées et insaturées. Ces impuretés étant souvent toxiques, des standards sévères sont susceptibles d'être adoptés limitant la teneur, par exemple, en impuretés oléfiniques à moins de 5 ppm en volume (Voir par exemple le projet de standard de la FDA (Food and Drug Administration, Center for Drug and Evaluation and Research, Octobre 1998) concernant la teneur en dites impuretés de 1,1,1,2-tétrafluoroéthane pour des produits MDI).

En conséquence il est nécessaire d'épurer l'hydrofluoroalcane de qualité industrielle afin d'obtenir un produit de qualité pharmaceutique. Il est également nécessaire de disposer de méthodes d'analyse permettant de détecter et d'identifier des traces d'impuretés organiques insaturées et saturées dans l'hydrofluoroalcane. Ceci pose des problèmes notamment lorsque ces impuretés ont un point d'ébullition proche de l'hydrofluoroalcane.

La demande de brevet WO-A-90/8750 concerne un procédé d'épuration de 1,1,1,2-tétrafluoroéthane en impuretés oléfiniques selon lequel on soumet le 1,1,1,2-tétrafluoréthane à une hydrogénation catalytique. Selon ce procédé connu, on observe des teneurs allant jusqu'à 10 ppm en une seule impureté oléfinique, à savoir le 1,1-difluoro-2-chloroéthylène. Selon la demande de brevet, la limite détection de 1,1-difluoro-2-chloroéthylène est de 10 ppm. De plus, ce procédé connu ne permet pas une épuration satisfaisante en toutes les impuretés organiques saturées et insaturées. Notamment, ce procédé connu ne permet pas d'éliminer le 1,1,2,2-tétrafluoroéthane (HFC-134). La présence de quantités substantielles de HFC-134 dans du 1,1,1,2-tétrafluoroéthane pour applications pharmaceutiques n'est pas souhaitable dès lors que sa toxicité a été peu examinée.

La méthode d'analyse selon l'invention s'applique aux hydrofluoroalcanes susceptibles d'être utilisés dans des applications pharmaceutiques. De tels hydrofluoroalcanes sont le 1,1,1,2-tétrafluoroéthane, le 1,1,1,2,3,3,3-heptafluoropropane, le 1,1,1,3,3-pentafluoropropane et le 1,1,1,3,3-pentafluorobutane de qualité pharmaceutique. La méthode d'analyse selon l'invention s'applique de manière préférée à l'analyse du 1,1,1,2-tétrafluoroéthane de qualité pharmaceutique.

L'hydrofluoroalcane présente une teneur individuelle en chaque impureté organique d'au plus 8 ppm. De manière préférée cette teneur individuelle est d'au plus 5 ppm. Une teneur individuelle d'au plus 2,8 ppm est particulièrement préférée. Une teneur individuelle d'au plus 1 ppm est encore plus préférée.

Les teneurs en impuretés dans l'hydrofluoroalcane peuvent être déterminées, de manière avantageuse, selon la méthode décrite dans l'exemple 4. L'hydrofluoroalcane est utilisable pour des applications pharmaceutiques.

La teneur totale en impuretés organiques insaturées dans l'hydrofluoroalcane est généralement d'au plus 5 ppm molaire. Souvent cette teneur est d'au plus 3 ppm. Plus souvent la teneur est d'au plus 2 ppm. De préférence la teneur est d'au plus 1,8 ppm. De manière particulièrement préférée, la teneur est d'au plus 1,7 ppm.

De préférence, l'hydrofluoroalcane est le 1,1,1,2-tétrafluoroéthane.

La teneur en fluoropropènes déterminée dans le 1,1,1,2-tétrafluoroéthane selon l'invention est généralement moins de 1,4 ppm molaire. Souvent cette teneur est d'au plus 1,2 ppm. Plus souvent, la teneur est d'au plus 1,0 ppm. De préférence la teneur est d'au plus 0,9 ppm. De manière particulièrement préférée, la teneur est d'au plus 0,8 ppm.

La teneur en 1,1,2,2-tétrafluoroéthane dans le 1,1,1,2-tétrafluoroéthane est généralement moins de 10 ppm molaire. De préférence la teneur est d'au plus 8 ppm. De manière particulièrement préférée, la teneur est d'au plus 5 ppm. De manière encore plus préférée, la teneur est d'au plus 2 ppm.

L'invention concerne une méthode d'analyse de la teneur en impuretés organiques d'un hydrofluoroalcane dans laquelle
(a) on soumet l'hydrofluoroalcane à une opération de chromatographie gazeuse dans laquelle la phase stationnaire comprend au moins un polyalkylsiloxane fonctionnalisé par des groupements polaires nitrile et ;
(b) on effectue une opération de détection des impuretés organiques par spectrométrie de masse dans laquelle la méthode de détection est sélectionnée parmi le mode ion ciblé ou le mode temps-de-vol.

La méthode selon l'invention permet, de manière surprenante de déterminer en une seule opération d'analyse la nature et la quantité d'un grand nombre d'impuretés organiques présentes dans un hydrofluoroalcane. La méthode selon l'invention permet même d'effectuer une détection quantitative de plusieurs impuretés organiques présentant entre elles le même temps de rétention dans l'opération de chromatographie. De manière particulièrement surprenante, la méthode selon l'invention permet également la détection quantitative d'impuretés qui présentent le même temps de rétention dans l'opération de chromatographie que l'hydrofluoroalcane.

II a également été trouvé que le HCFC-124 (1,1,1,2-tétrafluoro-2-chloroéthane) qui est une impureté pouvant être présente dans le 1,1,1,2,3,3,3-heptafluoropropane et qui ne pouvait pas être déterminé simultanément avec d'autres impuretés organiques préalablement à la présente invention, peut être analysé en une même opération avec les autres impuretés organiques grâce à la méthode selon l'invention.

L'opération de chromatographie est une opération de chromatographie gazeuse.La phase stationnaire dans l'opération de chromatographie comprend au moins un polyalkylsiloxane fonctionnalisé par des groupements polaires de type nitrile.

La phase stationnaire présente une polarité moyenne. Une telle phase stationnaire peut être composée, par exemple, par un mélange de polymère non-polaire tel qu'un polymère de type polysiloxane avec un polymère polaire. De tels polymères polaires sont choisis parmi les polymères fonctionnalisés par des groupements polaires, en particulier parmi les polyalkylsiloxanes fonctionnalisés. Le groupement polaire est un groupement de type nitrile. Un polysiloxane de formule générale 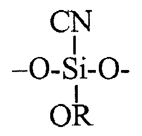 dans laquelle R est un groupement alkyle en C1 à C4, de préférence, un groupement méthyle est préféré à titre de polymère polaire.

La teneur en polymère polaire est généralement supérieure ou égale à 1 % en poids de la phase stationnaire. Souvent cette teneur est supérieure ou égale à 2 % en poids. De préférence, elle est supérieure ou égale à environ 5 % en poids. La teneur en polymère polaire est généralement inférieure ou égale à 15 % en poids de la phase stationnaire. Souvent la teneur est inférieure ou égale à 10 % en poids. De préférence, elle est inférieure ou égale à environ 8 % en poids.

La température initiale de l'opération de chromatographie est réglée au plus à 40°C. Souvent cette température est réglée au plus à 0°C. Plus souvent, cette température est réglée au plus à -20°C. De préférence, cette température est réglée au plus à -40°C. En règle générale, elle est d'au moins -80°C.

Dans l'opération de chromatographie on travaille généralement avec au moins une étape à gradient de température constant qui assure une hausse de température contrôlée au départ de la température initiale. Ce gradient de température est généralement d'au moins 0,1°C/min. De préférence il est d'au moins 0,5°C/min.

Le gradient de température est généralement d'au plus 10°C/min. De préférence il est d'au plus 2°C/min.

La colonne est, de préférence une colonne capillaire. La longueur de la colonne est généralement d'au plus 200 m. Souvent la longueur est d'au plus 120 m. La longueur de la colonne est généralement d'au moins 20 m.

L'injection peut être effectuée en mode « split » ou « splitless ». Une injection en mode « split » est préférée.

Le gaz porteur est souvent choisi parmi l'hélium et l'hydrogène. L'hélium est préféré.

Le diamètre intérieur de la colonne est généralement d'au plus 0,32 mm.

Souvent le diamètre est d'au plus 0,25 mm. De préférence, le diamètre est d'au plus 0,20 mm. Souvent le diamètre intérieur de la colonne est d'au moins 0,10 mm. De préférence, le diamètre est d'au moins 0,15 mm.

L'épaisseur du film de phase stationnaire déposé à l'intérieur de la colonne est généralement d'au moins 0,5µm. De préférence, l'épaisseur est supérieure ou égale à environ 1µm. L'épaisseur du film de phase stationnaire déposé à l'intérieur de la colonne est généralement d'au plus 5µm.

Une variante particulière de la méthode selon l'invention s'applique de manière préférée lorsque le diamètre intérieur et l'épaisseur du film se situent à l'intérieur des gammes préférées.

La longueur de la colonne est dans cette variante de manière avantageuse d'au moins 30m. De manière plus particulièrement préférée, elle est supérieure ou égale à environ 40m. La longueur de la colonne est de manière avantageuse d'au plus 100m. De manière plus particulièrement préférée, elle est inférieure ou égale à environ 60m.

Dans cette variante le gradient de température tel que décrit plus haut est généralement d'au moins 10°C/min. De préférence il est d'au moins 20°C/min. De manière plus particulièrement préférée le gradient est supérieur ou égal à environ 40°C. Le gradient de température dans cette variante est généralement d'au plus 50°C/min.

La température initiale dans cette variante est généralement d'au plus -10°C. De préférence, elle est inférieure ou égale à -20°C. La température initiale dans cette variante est généralement d'au moins -50°C.

Cette variante de la méthode selon l'invention permet, de manière surprenante, d'accélérer davantage l'opération d'analyse tout en conservant les autres avantages de la méthode selon l'invention, en particulier quant à la détection et détermination simultanée des impuretés organiques.

Des colonnes de chromatographie gazeuse préfabriquées permettant la mise en oeuvre de la méthode selon l'invention sont commercialement disponibles par exemple Rtx®-624 de la société Restec et DB®-624 de la société J & W.

La détection par spectrométrie de masse s'effectue en mode ion ciblé (selected ion monitoring (SIM)) ou en mode temps-de-vol (TOF). Des spectromètres de masse pour la détection en mode temps-de-vol préférés dans la méthode selon l'invention, permettent d'enregistrer un nombre élevé de spectre de masse par seconde, à savoir environ 1 à 500, de préférence 100 à 500 spectres par seconde. Des spectromètres utilisables pour mettre en oeuvre la méthode selon l'invention sont par exemple, ceux commercialisées par la société LECO Corporation sous le nom PEGASUS® II et ceux commercialisés par la société THERMOQUEST sous le nom TEMPUS™.

Les hydrofluoroalcanes dont la teneur en impuretés organiques peut être analysée par la méthode selon l'invention, sont le 1,1,1,2,3,3,3-heptafluoropropane, le 1,1,1,3,3-pentafluoropropane, le 1,1,1,3,3-pentafluorobutane et 1,1,1,2-tétrafluoroéthane dans lesquels une teneur individuelle de chaque impureté organique est d'au plus 8 ppm molaire. La méthode selon l'invention s'applique, de préférence, à l'analyse de 1,1,1,2-tétrafluoroéthane ou de 1,1,1,2,3,3,3-heptafluoropropane. Elle s'applique, en particulier, à l'analyse de 1,1,1,2-tétrafluoroéthane.

La méthode selon l'invention est particulièrement efficace, car la détermination de la teneur en l'ensemble des impuretés organiques peut être obtenue par une seule opération d'analyse. Dès lors seule cette opération doit être validée, c'est à dire étalonnée et vérifiée. Aussi la calibration éventuellement nécessaire entre l'analyse de différents échantillons est simplifiée.

La méthode selon l'invention permet d'atteindre une durée très courte nécessaire pour l'analyse, qui peut être effectuée typiquement en moins de deux heures, souvent en moins d'une heure. On peut arriver à une analyse complète des impuretés dans une durée d'environ 10 minutes. Cette efficacité permet en particulier d'améliorer la performance de procédés industriels de fabrication nécessitant un contrôle de qualité d'un hydrofluoroalcane. En effet il est possible de satisfaire avec plus de flexibilité et rapidité des commandes urgentes d'hydrofluoroalcane et de réduire les temps de stockage d'hydrofluoroalcane.

L'invention concerne dès lors aussi un procédé de fabrication d'un hydrofluoroalcane, comprenant l'utilisation de la méthode d'analyse selon l'invention pour contrôler la qualité de l'hydrofluoroalcane.

L'hydrofluoroalcane est un hydrofluoroalcane épuré.

Le procédé de fabrication d'un hydrofluoroalcane peut comprendre une étape d'épuration. De préférence, ce procédé comprend
(a) une utilisation de la méthode selon l'invention pour l'analyse d'un hydrofluoroalcane brut ;
(b) une épuration de l'hydrofluoroalcane brut pour obtenir un hydrofluoroalcane épuré ;
(c) et une seconde utilisation de la méthode selon l'invention pour l'analyse de l'hydrofluoroalcane épuré.

Le procédé de fabrication d'un hydrofluoroalcane selon l'invention s'applique aux hydrofluoroalcanes de qualité pharmaceutique cités plus haut.

L'invention concerne aussi un procédé de fabrication d'un aérosol pharmaceutique comprenant au moins un hydrofluoroalcane de qualité pharmaceutique, comprenant l'utilisation de la méthode d'analyse selon l'invention pour contrôler la qualité de l'hydrofluoroalcane de qualité pharmaceutique.

Le procédé de fabrication d'un aérosol pharmaceutique selon l'invention convient particulièrement pour la fabrication d'un aérosol pharmaceutique pour inhalation, comprenant au moins un hydrofluoroalcane liquéfié sous pression et un médicament. Le médicament est présent, de préférence sous la forme d'une poudre à l'état de suspension. L'hydrofluoroalcane est présent à titre de gaz propulseur.

Le procédé de fabrication d'un aérosol pharmaceutique est particulièrement intéressant, car la méthode d'analyse permet d'effectuer le contrôle de qualité sévère imposé pour des applications pharmaceutiques de manière particulièrement efficace.

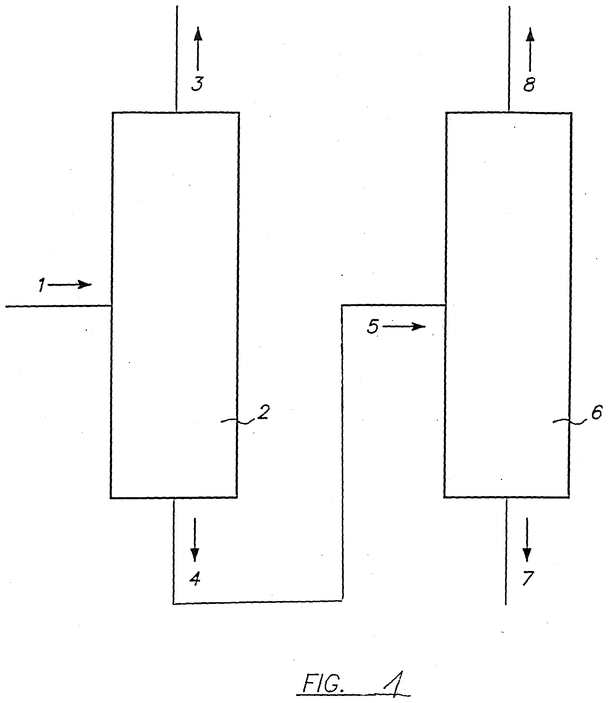

La figure 1 représente un schéma d'installation utilisable pour mettre en oeuvre unprocédé d'obtention d'un hydrofluoroalcane épuré (non conforme à l'invention). Les numéros font référence à la figure 1. L'hydrofluoroalcane contenant des impuretés organiques est introduit par la voie (1) dans une première colonne de distillation (2). En tête (3) de cette colonne (2) on obtient une fraction comprenant des impuretés légères (point d'ébullition inférieur mais voisin), et celles qui forment à cette pression un azéotrope à température minimum. En pied (4) de cette colonne (2) on obtient une fraction contenant de l'hydrofluoroalcane avec une teneur réduite en impuretés légères que l'on introduit par la voie (5) dans la deuxième colonne de distillation (6) fonctionnant à pression plus élevée que la colonne (2). On récupère, en pied (7) de la deuxième colonne (6), une fraction enrichie en impuretés lourdes qui présentent un point d'ébullition supérieur par rapport à celui de l'hydrofluoroalcane. En tête (8) de la deuxième colonne (6), on obtient de l'hydrofluoroalcane épuré en impuretés organiques.

Les exemples donnés ci-après entendent illustrer, de manière non limitative, le procédé et la méthode d'analyse selon l'invention.

### Exemple 1 (non conforme à l'invention)

On a mis en oeuvre une fraction impure de 1,1,1,2-tétrafluoroéthane contenant 57,2 ppm molaire d'impuretés organiques saturées et 10,6 ppm molaire d'impuretés organiques insaturées. On a introduit cette fraction au niveau du 7 ème plateau théorique dans une première colonne de distillation comprenant 20 plateaux théoriques. La pression dans la première colonne était de 6.75 bars abs. On a assuré un taux de reflux de 250. On a soutiré en tête une fraction correspondant à 10 % de l'alimentation et contenant 51,4 ppm molaire d'impuretés organiques saturées et 3,9 ppm molaire d'impuretés organiques insaturées. En pied on a récupéré une fraction constituée de 1,1,1,2-tétrafluoroéthane épuré en impuretés « légères » contenant 41,5 ppm molaire d'impuretés organiques saturées et 34.9 ppm d'impuretés organiques insaturées que l'on a introduit au niveau du 13 ème plateau dans une deuxième colonne de distillation contenant 20 plateaux théoriques. La pression de cette deuxième colonne de distillation était de 19,3 bars absolu. On a assuré un taux de reflux de 14,8. En pied de colonne on a soutiré 10 % de l'alimentation de la première colonne et contenant 320 ppm molaire d'impuretés organiques saturées 10 et 9,8 ppm molaire d'impuretés organiques insaturées. En tête de cette colonne on a soutiré une fraction constituée de 1,1,1,2-tétrafluoroéthane épuré en impuretés lourdes contenant 10,7 ppm molaire d'impuretés organiques saturées et 1,3 ppm molaire d'impuretés organiques insaturées.

### Exemple 2 (non conforme à l'invention)

On a mis en oeuvre une fraction impure de 1,1,1,2-tétrafluoroéthane contenant 2750 ppm molaire d'impuretés organiques saturées et 75 ppm molaire d'impuretés organiques insaturées. On a introduit cette fraction au niveau du 14 ème plateau théorique dans une première colonne de distillation comprenant 20 plateaux théoriques. La pression dans la première colonne était de 13 bars abs. On a assuré un taux de reflux de 50. On a soutiré en tête une fraction correspondant à 10 % de l'alimentation et contenant 1950 ppm molaire d'impuretés organiques saturées et 150 ppm molaire d'impuretés organiques insaturées. En pied on a récupéré une fraction constituée de 1,1,1,2-tétrafluoroéthane épuré en impuretés « légères » contenant 2950 ppm molaire d'impuretés organiques saturées et 40 ppm d'impuretés organiques insaturées que l'on a introduit au niveau du 14 ème plateau dans une deuxième colonne de distillation contenant 20 plateaux théoriques. La pression de cette deuxième colonne de distillation était de 12 bars absolu. On a assuré un taux de reflux de 12,5. En pied de colonne on a soutiré 10 % de l'alimentation de la première colonne et contenant 15500 ppm molaire d'impuretés organiques saturées et 50 ppm molaire d'impuretés organiques insaturées. En tête de cette colonne on a soutiré une fraction constituée de 1,1,1,2-tétrafluoroéthane épuré en impuretés lourdes contenant 275 ppm molaire d'impuretés organiques saturées et 31 ppm molaire d'impuretés organiques insaturées.

### Exemple 3 (non conforme à l'invention)

On a mis en oeuvre une fraction impure de 1,1,1,2-tétrafluoroéthane contenant 2950 ppm molaire d'impuretés organiques saturées et 50 ppm molaire d'impuretés organiques insaturées. On a introduit cette fraction au niveau du 14 ème plateau théorique dans une première colonne de distillation comprenant 20 plateaux théoriques. La pression dans la première colonne était de 7,5 bars abs. On a assuré un taux de reflux de 50. On a soutiré en tête une fraction correspondant à 10 % de l'alimentation et contenant 1830 ppm molaire d'impuretés organiques saturées et 200 ppm molaire d'impuretés organiques insaturées. En pied on a récupéré une fraction constituée de 1,1,1,2-tétrafluoroéthane épuré en impuretés « légères » contenant 3200 ppm molaire d'impuretés organiques saturées et 32 ppm d'impuretés organiques insaturées que l'on a introduit au niveau du 14 ème plateau dans une deuxième colonne de distillation contenant 20 plateaux théoriques. La pression de cette deuxième colonne de distillation était de 6,5 bars absolu. On a assuré un taux de reflux de 12,5. En pied de colonne on a soutiré 10 % de l'alimentation de la première colonne et contenant 27500 ppm molaire d'impuretés organiques saturées et 50 ppm molaire d'impuretés organiques insaturées. En tête de cette colonne on a soutiré une fraction constituée de 1,1,1,2-tétrafluoroéthane épuré en impuretés lourdes contenant 200 ppm molaire d'impuretés organiques saturées et 28 ppm molaire d'impuretés organiques insaturées.

### Exemple 4 (conforme à l'invention)

On a effectué une analyse de 1,1,1,2-tétrafluoroéthane de qualité pharmaceutique obtenu selon un procédé d'obtention conforme à l'invention. Pour ce faire, on a effectué une chromatographie gazeuse sur une colonne de chromatographie gazeuse Rtx®-1 commercialisée par la société RESTEK Corp, comprenant à titre de phase stationnaire 100 % de diméthylpolysiloxane réticulé par le procédé Crossbond®. Les dimensions de la colonne étaient 105m x 0.25 mm x 1,0 micron. On a utilisé un programme de température comprenant 2 étapes, la première commençant à -50°C et on a fait monter la température à une vitesse de 1°C/min jusqu'à une température de 0°C. Ensuite, dans une deuxième étape on a fait monter la température à une vitesse de 10°C/min jusqu'à 250°C. La détection a été effectuée par spectrométrie de masse en mode ciblage sur ion caractéristique (selected ion monitoring (SIM)) en utilisant un spectromètre de masse HP 5972 commercialisé par la société Hewlett Packard. On a effectué un étalonnage en mettant en oeuvre un mélange étalon gazeux constitué de 10 ppm de chaque impureté à analyser.

Le tableau ci-après montre la teneur en différentes impuretés du 1,1,1,2-tétrafluoroéthane selon l'invention et du 1,1,1,2-tétrafluoroéthane brut qui a été mis en oeuvre dans le procédé selon l'invention. La limite de détection de la méthode d'analyse selon l'invention est également indiquée. Cette limite de détection a été validée par un étalonnage statistique sur 5 concentrations de 1 à 10 ppm en chaque impureté individuelle. Les valeurs obtenues ont été corrigées en fonction de la pureté de chacune des impuretés présentes dans le mélange d'étalonnage.

Le tableau reprend en plus les valeurs contenues dans le projet de standard de FDA cité plus haut. Toutes les valeurs sont exprimées en ppm molaire, hormis la valeur « assay » qui est exprimée en pourcent. Un champ vide signifie que l'impureté en question n'a pas été observée.

| **Impureté organique** | **Formule** | **Limite de Détection de la méthode selon l'invention** | **Projet de Standard FDA** | **HFC-134a Brut** | **HFC-134a Invention** |
|---|---|---|---|---|---|
| **HFC-23** | CHF3 | 0.2 | 5 | | |
| **CFC-13** | CClF3 | 0.3 | 5 | | |
| **HFC-32** | CH2F2 | 0.2 | 5 | | |
| **HFC-125** | CHF2-CF3 | 0.3 | 5 | 50 | |
| **HFC-143a** | CH3-CF3 | 0.4 | 10 | 124 | |
| **CFC-115** | CClF2-CF3 | 0.2 | 5 | 0.3 | |
| **HFC-1123** | CHF=CF2 | 0.2 | 5 | | |
| **HFC**-**1318my**-**c**/**t** | CF3-CF-CF-CF3 | 0.7 | 5 | | |
| **HFC**-**1318my**-**t**/**c (1)** | CF3-CF-CF-CF3 | 0.3 | 5 | < | < |
| **HFC-245cb** | CF3-CF2-CH3 | 0.2 | 5 | 0.3 | |
| **HFC**-**1234yf** | CH2=CF-CF3 | 0.3 | 5 | 1.3 | |
| **HFC-134** | CHF2-CHF2 | 0.4 | 1000 | 2824 | < |
| **HFC-152a** | CH3-CHF2 | 0.2 | 300 | 1.3 | |
| **HFC**-**217ba** | CF3-CCIF-CF3 | 0.5 | 5* | 2.4 | 0.5 |
| **HFC-161** | CH3-CH2F | 0.2 | 30 | | |
| **HFC-1225ye** | CHF-CF-CF3 | 0.2 | 5 | 1.8 | |
| **HFC-1243zf** | CH2=CH-CF3 | 0.4 | 5 | 7.3 | 0.9 |
| **HFC-1132** | CHF=CHF | 0.4 | 5 | < | |
| **C3H2F4 (2)** | | 0.3 | 5* | 11.8 | |
| **HCFC-22** | CHClF2 | 0.2 | 50 | 0.2 | |
| **HFC-1336mzz** | CF3-CH-CH-CF3 | 0.5 | 5 | | |
| **CFC-12** | CCl2F2 | 0.2 | 100 | 0.2 | |
| **HCC-40** | CH3Cl | 0.4 | 5 | 4.0 | |
| **HCFC-124a** | CHF2-CClF2 | 0.2 | 5 | | |
| **HCFC-124** | CHClF-CF3 | 0.4 | 100 | 36 | |
| **HCFC-1122** | CHCl=CF2 | 0.3 | 5 | 28 | 0.3 |
| **HCFC-31** | CH2ClF | 0.4 | 5 | 0.4 | |
| **CFC-114** | CClF2-CClF2 | 0.1 | 5 | | |
| **CFC-114a** | CC12F-CF3 | 0.1 | 25 | 19 | |
| **HFC-152** | CH2F-CH2F | 0.5 | 5 | | |
| **HCFC-1122a c/t (3)** | CHF-CClF | 0.3 | 5* | 4.6 | |
| **HCFC-1122a c/t (3)** | CHF-CClF | 0.3 | 5* | < | |
| **HCFC-133a** | CH2Cl-CF3 | 0.4 | 5 | 0.4 | |
| **HCFC-1131trans** | CHCl=CHF | 0.5 | 5* | 10.3 | 0.2 |
| **HCFC**-**1131 cis** | CHCl=CH | 0.5 | 5* | | |
| **CFC-12B1** | CClBrF2 | 0.2 | 5 | | |
| **CFC-1112a** | CF2=CCl2 | 0.3 | 5 | | |
| **HCFC-123** | CHCl2-CF3 | 0.4 | 5 | | |
| **CFC**-**11** | CC13F | 0.3 | 5 | | |
| **HCFC-123a** | CHClF-CClF2 | 0.3 | 5 | | |
| **HCFC-1121 c/t** | CHCl-CClF | 0.2 | 5 | | |
| **HCC-30** | CH2Cl2 | 0.6 | 5* | < | |
| **HCFC-132b** | CClF2-CH2Cl | 0.6 | 5 | | |
| **CFC-113** | CClF2-CCl2F | 0.4 | 5 | | |
| **HCC-1120** | CHCl=CCl2 | 0.8 | 5* | < | < |
| **Somme d'impuretés organiques** | | | **1000** | **3127.6** | **1.9** |
| **Somme d**'**oléfines** | | | **5** | **65.1** | **1.4** |
| **Assay** | | | **99**.**9** % | | **100** % |

| | | | | | |
|---|---|---|---|---|---|
| < inférieur à la limite de détection Les impuretés dont la teneur est inférieure à la limite de détection n'ont pas été prises en compte pour le calcul des sommes d'impuretés. (1) valeur estimée (2) valeur calculée en prenant en compte le facteur de réponse du HFC-1234yf (3) isomères E/Z, valeur calculée en prenant en compte le facteur de réponse du HCFC-1122 * Composé non cité individuellement dans le projet de standard FDA. | | | | | |

Il apparaît que le 1,1,2,2-tétrafluoroéthane présente une teneur en impuretés organiques, extrêmement basse et largement inférieure aux valeurs du projet de standard FDA. De plus, la teneur d'aucune impureté organique individuelle ne dépasse 1 ppm molaire.

Il apparaît également que la méthode permet de détecter et d'identifier, avec une sensibilité extrêmement élevée toutes les impuretés incluses dans le projet de standard de la FDA.

## Revendications

1. Méthode d'analyse de la teneur en impuretés organiques d'un hydrofluoroalcane de qualité pharmaceutique sélectionné parmi le 1,1,1,2,3,3,3-heptafluoropropane, le 1,1,1,3,3-pentafluoropropane, le 1,1,1,3,3-pentafluorobutane et le 1,1,1,2-tétrafluoroéthane dans laquelle l'hydrofluoroalcane présente une teneur individuelle de chaque impureté organique d'au plus 8 ppm molaire, et dans laquelle
a) on soumet l'hydrofluoroalcane à une opération de chromatographie gazeuse dans laquelle la phase stationnaire comprend au moins un polyalkylsiloxane fonctionnalisé par des groupements polaires de type nitrile et ;
b) on effectue une opération de détection des impuretés organiques par spectrométrie de masse dans laquelle la méthode de détection est sélectionnée parmi le mode ion ciblé (selected ion monitoring (SIM)) ou le mode temps-de-vol (time of flight (TOF)).

2. Méthode selon la revendication 1, dans laquelle la température initiale de l'opération de chromatographie est réglée au plus à 40°C.

3. Méthode selon la revendication 2, dans laquelle la température initiale de l'opération de chromatographie est inférieure ou égale à environ 20°C.

4. Méthode selon l'une quelconque des revendications 1 à 3, dans laquelle l'hydrofluoroalcane est le 1,1,1,2-tétrafluoroéthane.

5. Procédé de fabrication d'un hydrofluoroalcane, comprenant l'utilisation de la méthode selon l'une quelconque des revendications 1 à 4 pour contrôler la qualité de l'hydrofluoroalcane.

6. Procédé de fabrication d'un aérosol pharmaceutique comprenant au moins un hydrofluoroalcane de qualité pharmaceutique comprenant l'utilisation de la méthode selon l'une quelconque des revendications 1 à 4 pour contrôler la qualité de l'hydrofluoroalcane de qualité pharmaceutique.

## Patentansprüche

1. Verfahren zur Analyse des Gehalts an organischen Verunreinigungen eines Hydrofluoralkans pharmazeutischer Qualität ausgewählt aus 1,1,1,2,3,3,3-Heptafluorpropan, 1,1,1,3,3-Pentafluorpropan, 1,1,1,3,3-Pentafluorbutan und 1,1,1,2-Tetrafluorethan, wobei das Hydrofluoralkan einen individuellen Gehalt von höchstens 8 Mol-ppm an einer jeden organischen Verunreinigung aufweist, und wobei
a) man das Hydrofluoralkan einem Gaschromatographieschritt aussetzt, wobei die stationäre Phase mindestens ein durch polare Gruppen vom Nitril-Typ funktionalisiertes Polyalkylsiloxan beinhaltet, und;
b) man einen Schritt zur Detektion der organischen Verunreinigungen mittels Massenspektrometrie durchführt, wobei das Detektionsverfahren ausgewählt ist aus dem Verfahren der Einzelmassenregistrierung ("selected ion monitoring (SIM)") oder dem Verfahren der Flugzeitbestimmung ("time of flight (TOF)").

2. Verfahren gemäß Anspruch 1, wobei die Ausgangstemperatur des Chromatographieschritts auf höchstens 40°C eingestellt ist.

3. Verfahren gemäß Anspruch 2, wobei die Ausgangstemperatur des Chromatographieschritts weniger als oder gleich ungefähr 20°C beträgt.

4. Verfahren gemäß einem beliebigen der Ansprüche 1 bis 3, wobei das Hydrofluoralkan 1,1,1,2-Tetrafluorethan ist.

5. Verfahren zur Herstellung eines Hydrofluoralkans, umfassend die Verwendung des Verfahrens gemäß einem beliebigen der Ansprüche 1 bis 4 zur Qualitätskontrolle des Hydrofluoralkans.

6. Verfahren zur Herstellung eines pharmazeutischen Aerosols umfassend mindestens ein Hydrofluoralkan pharmazeutischer Qualität umfassend die Verwendung des Verfahrens gemäß einem beliebigen der Ansprüche 1 bis 4 zur Qualitätskontrolle des Hydrofluoralkans pharmazeutischer Qualität.

## Claims

1. Method for analysis of the content of organic impurities of a hydrofluoroalkane of pharmaceutical quality selected from 1,1,1,2,3,3,3-heptafluoropropane, 1,1,1,3,3-pentafluoropropane, 1,1,1,3,3-pentafluorobutane and 1,1,1,2-tetrafluoroethane in which the hydrofluoroalkane has an individual content of each organic impurity of at most 8 ppm molar, and in which
a) the hydrofluoroalkane is subjected to a gas chromatography operation in which the stationary phase comprises at least one polyalkylsiloxane functionalised by polar groups of nitrile type and;
b) an operation for detection of organic impurities is performed by mass spectrometry in which the detection method is selected from selected ion monitoring (SIM)) type or time of flight (TOF)) type.

2. Method according to claim 1, in which the initial temperature of the chromatography operation is set at 40°C at the highest.

3. Method according to claim 2, in which the initial temperature of the chromatography operation is less than or equal to approximately 20°C.

4. Method according to any one of claims 1 to 3, in which the hydrofluoroalkane is 1,1,1,2-tetrafluoroethane.

5. Process for manufacture of a hydrofluoroalkane, comprising the use of the method according to any one of claims 1 to 4 to monitor the quality of the hydrofluoroalkane.

6. Process for manufacture of a pharmaceutical aerosol comprising at least one hydrofluoroalkane of pharmaceutical quality comprising the use of the method according to any one of claims 1 to 4 to monitor the quality of the hydrofluoroalkane of pharmaceutical quality.
